# EUROPEAN PATENT APPLICATION

(11) **EP 3 805 361 A1**
(43) Date of publication of application: **14.04.2021**
(21) Application number: 19814013.9
(22) Date of filing: 10.05.2019
(51) Int. Cl.: C12M 3/00

(54) **MICRODROPLET TREATMENT DEVICE AND USE METHOD THEREOF**

(30) Priority: 07.06.2018 CN 201810581405
(71) Applicant: Luoyang TMAXTREE Biotechnology Co., Ltd, Luoyang, Henan 471023 (CN)
(72) Inventor: WANG, Liyan, Luoyang, Henan 471023 (CN); DUAN, Baofeng, Luoyang, Henan 471023 (CN); GUO, Xiaojie, Luoyang, Henan 471023 (CN)
(74) Representative: Lavoix
(86) International application number: PCT/CN2019/086430
(87) International publication number: WO 2019/233245

(57) **Abstract**

A microdroplet treatment device, comprising: a sample introduction system, a microfluidic chip system (46), a temperature control system, a droplet recognition system, a droplet detection system and a control system (45); the sample introduction system is used for introducing, into the microdroplet treatment device, samples of aqueous phase and oil phase, and the sample introduction system comprises at least: a sample introduction system I (41) and a sample introduction system II (42), the sample introduction system I (41) being used for introducing, into the microdroplet treatment device, samples of oil phase, and the sample introduction system II (42) being used for introducing into the microdroplet treatment device, samples of aqueous phase; the microfluidic chip system (46) comprises a substrate (4), pipes (1a, 1b, 1c, 1d, 2, 3) formed in the substrate, and a first detection window (9) and a second detection window (10); the temperature control system comprises: a temperature sensor and a temperature control member; the droplet recognition system comprises: a laser light source (55) and a photoelectric sensor (56); the droplet detection system comprises: an optical fiber (57), a spectrometer (58) and a halogen light source (59); and the control system (45) is used for controlling each of the systems in the microdroplet treatment device.

## Description

### TECHNICAL FIELD

The invention belongs to the field of microfluidics, and especially relates to a microdroplet treatment device and use method thereof. Particularly, the microdroplet treatment device may be a microbial droplet culture device.

### BACKGROUND ART

Microfluidic chip technology is widely used for sample preparation, reaction, separation, and detection in the process of biological, chemical, and medical analysis etc. It is one of the rapidly developing frontier technologies and the most active research fields. The microfluidic chip for processing a sample solution has high requirements for sterilization and the stability of sample introduction. The sample introduction of the microfluidic chip needs to be slow, such as pL-nL/s level. Droplet microfluidics has higher requirements for the stability of sample introduction, and slight fluctuations will affect the stability and uniformity of the droplets.

Traditional microbial breeding generally adopts plate coating culture to obtain single colony, then verifying by scale fermentation culture in shaking flask. Since usually it will take several months to several years for ordinary microorganisms to go through the process of preliminary screening, re-screening, and being confirmed to meet the production needs. This selection method needs a long screening cycle. Secondly, for traditional breeding methods, the fermentation culture at the level of shaking flask and the evaluation process needs enough manpower, experiment space, and culture space, and the screening efficiency may only reach 10¹⁻²/batch, resulting in low screening throughput; thirdly, since the success of mutagenesis screening is closely related to the number of screening, a large amount of screening samples are required for obtaining mutants with a target trait, thereby making the workload of R&D staff very heavy. Finally, traditional breeding methods are based on solid culture or large-volume liquid culture, resulting in a large consumption of materials and resources for culture and detection, and this brings high experimental cost.

In order to solve the above problems, the screening technology of microplate culture and microfluidic technology have been developed. The screening technology of microplate reduces the culture system from 50-100ml at the level of shaking flask to several milliliters to tens of microliters, and may simultaneously cultivate multiple samples such as 24, 48, 96, 384, and 1536 samples, etc., and then the online monitoring of certain process parameters may be realized by combining with corresponding automatic monitoring equipment. In order to improve the screening efficiency of microplates, professional supporting equipment including automatic equipment for monoclonal selection, automatic equipment for sterilization medium preparation, automatic medium-dispensing system, and automatic instrument for bacterial plate dilution have been developed for the multi-well plate system, thereby greatly improving the work efficiency.

Microfluidic technology was developed in the field of analytical chemistry in the 1990s. It is a miniature analyzing experiment equipment for integratedly realizing the preparation, introduction, reaction, separation, and detection of micro-samples based on a micro-pipe network structure. Microfluidic technology has extremely high efficiency. Because of the small structure, it is easy to integrate hundreds of microbial culture units on a chip at one time, thereby saving a lot of culture medium. The operation of the entire experimental process may be simulated on a chip by integrated operation with software.

Patent document CN103983794A disclosed a microfluidic chip in 2014, which comprises a detection window (detection hole), an electrode, a bifurcation structure-containing channel, a liquid inlet pool (or liquid inlet interface), finished product pool (or liquid outlet interface), waste liquid pool (or waste liquid interface), etc., wherein the liquid inlet pool (or liquid inlet interface), finished product pool (or liquid outlet interface), waste liquid pool (or waste liquid interface) are physically connected with the channel in respect, the detection window (or detection hole) is located near the channel, and the electrodes are fixed on both sides of the channel close to the bifurcation structure, thereby structurally solving the five problems of the identification of droplet position, the control of droplet volume, the segmentation of a single droplet sample, the separation of multiple samples in a droplet, and the selection of droplet parameters in microfluidic, then the material carrier of microfluidic technology is realized, but the detection function cannot be realized, and the cultivation and screening of microbial droplets still cannot be controlled online.

Patent document CN104007091A disclosed a high-throughput detection system based on a droplet microfluidic chip in 2014, which mainly comprises a droplet microfluidic chip system, an optical path system, and a data acquisition and analysis system; wherein the droplet microfluidic chip system embeds the microorganisms to be detected to form an independent single-droplet micro-reaction chamber, and the laser-induced fluorescence detection signal of a microbial sample in the single-droplet micro-reaction chamber is transmitted through the optical path system, and the collected signal is detected and analyzed by the data acquisition and analysis system through computer software, thereby realizing efficient screening of target enzymes and metabolites related to the production of different types of industrial microorganisms such as *E. coli, Corynebacterium glutamicum* and *Saccharomyces cerevisiae,* but it still cannot realize online culture of microorganisms.

Since the selection of microorganisms is a special biological process, firstly the microorganisms are inoculated and cultivated, and then they are tested and evaluated, finally the target microorganisms are selected. Therefore, there is a need for a new device to replace traditional inoculation, shaking flask culture, and traditional detection, as an equipment for realizing micro-volume, high-throughput and long-term continuous culture, real-time online detection, and conducting microbial breeding based on the growth performance and traits.

### CONTENTS OF THE INVENTION

In order to solve the above-mentioned problems, the present invention provides a microdroplet treatment device, which may be used for the cultivation of microbial droplets. The device may load hundreds of microdroplets containing microbes with a volume of 0.5-10 µL through a microfluidic chip, and complete microbial breeding through continuous online culture, detection and sorting.

The object of the present invention is achieved through the following technical solutions.
1. A microdroplet treatment device, comprising: a sample introduction system, a microfluidic chip system, a temperature control system, a droplet recognition system, a droplet detection system and a control system; wherein
   the sample introduction system is used for introducing samples of aqueous phase and oil phase into the microdroplet treatment device, and the sample introduction system comprises at least: a sample introduction system I and a sample introduction system II, the sample introduction system I is used for introducing samples of oil phase into the microdroplet treatment device, and the sample introduction system II is used for introducing samples of aqueous phase into the microdroplet treatment device;
   the microfluidic chip system comprises a substrate, pipes formed in the substrate, a first detection window and a second detection window;
   the temperature control system comprises a temperature sensor and a temperature control component;
   the droplet recognition system comprises a laser light source and a photoelectric sensor;
   the droplet detection system comprises an optical fiber spectrometer and a halogen light source; and
   the control system is used for controlling each of the systems in the microdroplet treatment device.
2. The device according to item 1, wherein
   the droplet recognition system recognizes the sample droplets of aqueous phase passing through the first detection window of the microfluidic chip system by using the photoelectric sensor and the laser light source;
   the droplet detection system detects the spectral signal of the sample droplets of aqueous phase passing through the second detection window of the microfluidic chip system by using the optical fiber spectrometer and the halogen light source.
3. The device according to item 1 or 2, wherein
   the sample introduction system at least comprises two sample introduction systems I and one sample introduction system II.
4. The device according to item 3, wherein
   the sample introduction system comprises three sample introduction systems I and three sample introduction systems II.
5. The device according to item 3 or 4, wherein
   the sample introduction system I comprises a liquid container and a power source;
   the sample introduction system II comprises a liquid container, a power source, and a buffer bottle;
   the power source is a injection pump, a peristaltic pump, a diaphragm pump, and/or a plunger pump, preferably the injection pump;
   the liquid container contains a liquid immiscible and incompressible with the sample solution to be introduced;
   the power source drives the liquid entering the buffer bottle via an input pipe;
   the buffer bottle contains the sample solution, when the power source drives the liquid, it enters the buffer bottle via the input pipe to push the sample solution into the microfluidic chip system via an output pipe, and wherein the liquid container, the power source and the buffer bottle are connected via a capillary pipe.
6. The device according to any one of items 1-5, wherein
   the microfluidic chip system comprises:
   the substrate; and
   a first pipe, a second pipe, and a third pipe formed in the substrate, wherein
   the first pipe comprises a first connection port and a second connection port at both ends,
   the second pipe comprises a third connection port at one end, and communicates with the first pipe at the other end, and
   the third pipe comprises a fourth connection port at one end, and communicates with the first pipe at the other end,
   the first communication part of the first pipe and the second pipe is located upstream of the second communication part of the third pipe and the first pipe in the direction of droplet movement, and the distance between the first communication part and the second communication part is 500 µm-2000 µm, preferably 750 µm-1800 µm, more preferably 1000 µm-1500 µm,
   the first detection window and the second detection window formed on the first pipe,
   the first detection window and the second detection window are transparent areas formed on the first pipe, and
   a capillary pipe hermetically connected with the first pipe, the second pipe, and the third pipe.
7. The device according to item 6, wherein
   the first pipe is branched into a first pipe **a,** a first pipe **b,** a first pipe **c,** and a first pipe **d** upstream of the first communication part,
   wherein the first detection window and the second detection window are formed on the first pipe **a,**
   the first connection port and the second connection port on the first pipe **a,** the first pipe **b,** the first pipe **c,** and the first pipe **d** are hermetically connected with the capillary pipe,
   the second pipe and the third pipe are hermetically connected to the capillary pipe through the third connection port and the fourth connection port respectively.
8. The device according to item 6 or 7, wherein
   the substrate and the first pipe, the second pipe and the third pipe are made of glass, polymethylmethacrylate (PMMA), polycarbonate (PC), polystyrene (PS), or acrylonitrile-butadiene-styrene copolymer (ABS); preferably polymethylmethacrylate (PMMA);
   the capillary pipe is a rigid tube, and more preferably the capillary pipe is any one selected from the group consisting of: a polytetrafluoroethylene tube (PTFE tube), a copolymer tube of perfluoropropyl perfluorovinyl ether and polytetrafluoroethylene (PFA tube), a polyether ether ketone tube (PEEK tube), a polycarbonate tube (PC tube), and a polystyrene tube (PS tube); and
   more preferably the range of the cross-sectional area of the first pipe, the second pipe, the third pipe and the capillary pipe is 2.5×10⁻³ mm²-4 mm², preferably 0.01-3 mm², more preferably 0.1-2.5 mm², still more preferably 0.25-1 mm².
9. The device according to any one of items 1-8, wherein the sample introduction system and the microfluidic chip system are connected by capillary pipes and connection ports to form a hermetic closed-loop control structure.
10. The device according to item 1, wherein
   the sample introduction system, the microfluidic chip system, the droplet recognition system, and the droplet detection system are all provided in a box, and
   the temperature inside the box is controlled by the temperature control system,
   preferably the temperature inside the box is controlled within the range of 10°C-50°C, and the range of temperature fluctuation is controlled within ±0.5°C.
11. The device according to item 10, wherein the box further comprises a sterilization device, preferably the sterilization device is an ultraviolet lamp.
12. The device according to any one of items 1-11, wherein
   the control system comprises a controller and a PC display control system;
   the controller is respectively connected to the sample introduction system, the temperature control system, the droplet recognition system, and the droplet detection system, and takes control via a digital circuit in the control system;
   the PC display control system displays, stores and analyzes the information from the sample introduction system, the microfluidic chip system, the temperature control system, the droplet recognition system, and the droplet detection system.

The invention relates to a microbial droplet culture device, which comprises a sample introduction system, a microfluidic chip system, a temperature control system, a droplet recognition and detection system, and a control system, and is characterized in that:
the sample introduction system consists of a sample introduction system I and a sample introduction system II, wherein the sample introduction system I is used for introducing samples of oil phase, and the sample introduction system II is used for introducing samples of aqueous phase;
the microfluidic chip system consists of a substrate, microchannels formed on the substrate, and a detection window;
the cultivation system consists of pipelines connected to the microfluidic chip;
the temperature control system consists of a temperature sensor and a temperature control component;
the droplet recognition and detection system comprises an optical fiber spectrometer, a photoelectric sensor, a laser light source, a halogen light source etc., wherein the photoelectric sensor is used in conjunction with the laser light source to recognize the state of a microdroplet at the first detection window of the microfluidic chip system; and the optical fiber spectrometer is used in conjunction with the halogen light source to detect a sample in the accommodated microdroplet at the second detection window of the microfluidic chip system;
the control system comprises a controller and a PC display control system; wherein the controller is respectively connected to the sample introduction system, the temperature control system, and the detection system, and controls the digital circuit; and the PC display control system displays, stores and analyzes the information from the sample introduction system, the microfluidic chip system, the temperature control system, and the detection system.

The sample introduction system at least comprises two oil phase sample introduction systems and one aqueous phase sample introduction system; the sample introduction system may comprise multiple oil phase sample introduction systems and multiple aqueous phase sample introduction systems; the sample introduction power source is a pressure pump or a injection pump;
the microfluidic chip system may realize operations including the generation, segmentation, fusion, detection and separation of microdroplets;
the culture system consists of a soft hollow pipeline, one end of the pipeline is connected to the microchannel of the chip, and the other end of the pipeline may be connected to the microchannel of the chip or directly connected to the power device; in the device the microdroplets may flow from the chip to the pipeline, or from the pipeline to the chip; the pipeline of the culture system may be air-permeable or air-impermeable; the size range of inner diameter of the pipeline used in the culture system is 0.1-2mm;
after the microdroplets containing microorganisms are cultured, quantitative segmentation may be achieved on the chip, the segmented microdroplets may be fused with new microdroplets, and the fused new microdroplets may continue to be cultured; before or after the segmentation and fusion process, the microdroplets may be detected;
the sample introduction system, the microfluidic chip system, the culture system and the accessory power device are connected through pipes, interfaces, etc. to form a hermetic aseptic structure;
the sample introduction system, the microfluidic chip system, and the culture system are all placed in a temperature-controllable box, and the temperature inside the box is realized by a temperature control system; wherein the temperature control system consists of a temperature sensor and a temperature control component; and the temperature of the temperature control component may reach up to 50°C, the lowest temperature may reach 10°C, and the range of temperature fluctuation is controlled within ±0.5°C.

The temperature-controllable box comprises a sterilization device, wherein the sterilization device is an ultraviolet lamp.

The use method of the microbial droplet culture device comprises:
1) turning on the power of the device, and turning on the PC display control system;
2) exhausting the pipeline of the device, turning on the temperature control system, and preheating for 5-30 minutes;
3) initializing the pipeline liquid, the optical fiber spectrometer, and the photoelectric sensor by using the PC display control system;
4) selecting function modes, such as selecting the droplet generation, droplet segmentation and fusion, droplet culture, droplet sorting and other programs in the microfluidic chip system, setting the device parameters, and starting the operation;
5) obtaining the target droplets and exporting the data.

The microorganisms may be cultured continuously for at most 90 days according to the present invention.

The technical effects of the present invention are as follows:
Through the sample introduction system and the microfluidic chip system, it is possible to control the generation of 1-500 microdroplets in the microdroplet treatment device, and the volume of each microdroplet is 0.5-10µL. Using such microdroplet treatment device brings much higher throughout than shaking flasks and deep-well plates, and the medium consumption is less. The control system may also realize regular and quantitative replacement of fresh medium and addition of chemical factors, and this is far more convenient than traditional subculture by using seed liquid, thereby saving time and effort. The detection system may detects the growth of microorganisms in each microdroplet in real-time online, and it is far more convenient than traditional sample introduction and detection, thereby really accomplishing uninterrupted culture; it may also perform intelligent screening, set screening characteristics, and automatically screen suitable strains to improve effectiveness.

### BRIEF DESCRIPTION OF THE DRAWINGS

Fig. 1 is a functional schematic diagram of the microdroplet treatment device according to the present invention;
Fig. 2 is a perspective view of an embodiment of the microdroplet treatment device according to the present invention;
Fig. 3 shows a functional schematic diagram of a microfluidic chip used in the microdroplet treatment device according to the present invention;
Fig. 4 shows a schematic diagram of the microfluidic chip system used in the microdroplet treatment device according to the present invention;
Fig. 5 is a schematic diagram of the structure of the microfluidic chip according to the present invention which is used for the quantitative segmentation and fusion of microdroplets;
Fig. 6 is a schematic diagram of the structure of the connection of the power source and valve which is involved in the microfluidic chip system and the microdroplet treatment device according to the present invention;
Fig. 7 is a schematic diagram of the sample introduction system I according to the present invention;
Fig. 8 is a schematic diagram showing the connection of the chip system, the power source and the valve when it is used in the microdroplet treatment device according to the present invention;
Fig. 9 is a diagram showing the temperature change of the microdroplet under temperature control in the present invention;
Fig. 10 is a 46-hour *E. coli* growth curve determined by the microbial droplet culture device according to the present invention.

### Description of the Symbols:

1 (**1a**, **1b**, **1c**, **1d**) the first pipe; 2 the second pipe; 3 the third pipe; 4 substrate; 5 electrode; 6 electrode; 7 hole; 8 hole; 11 (11', 11", 11"') the first connection port; 12 the second connection port; 21 the third connection port; 31 the fourth connection port; 13 the first communication part; 14 the second communication part; 15 branching part;
41 sample introduction system I; 42 sample introduction system II; 43 temperature control system; 44 detection system (comprising droplet recognition system and droplet detection system); 45 control system; 46 microfluidic chip system; 47 container bottle **a;** 48 sample introduction power source **a;** 49 container bottle **b;** 50 sample introduction power source **b;** 51 container bottle **c;** 52 EP tube; 53 metal bath; 54 temperature controller; 55 laser light source; 56 photoelectric sensor; 57 optical fiber; 58 spectrometer; 59 halogen light source; 60 oil bottle; 61 sample introduction buffer bottle; 62 waste liquid bottle; 63 injection pump; 64 cooling fan; 65 rotary valve; 66 ultraviolet lamp; 67 temperature probe; 68 lighting lamp.

### SPECIFIC EMBODIMENTS

Hereinafter, specific examples of the present invention will be described in more detail with reference to the accompanying drawings. Although specific examples of the present invention are shown in the drawings, it should be understood that the present invention may be implemented in various forms and should not be limited by the examples set forth herein. On the contrary, these examples are provided to enable a more thorough understanding of the present invention and to fully convey the scope of the present invention to those skilled in the art.

It should be noted that certain words are used in the specification and claims to refer to specific components. Those skilled in the art should understand that they may use different terms to refer to the same component. This specification and claims do not use differences in terms as a way to distinguish components, but use differences in functions of components as a criterion for distinguishing. For example, "comprise" or "include" mentioned in the entire specification and claims is an open-ended term, and it should be interpreted as "including but not limited to". The following description of the specification is a preferred embodiment for implementing the present invention, but the description is based on the general principles of the specification and is not intended to limit the scope of the present invention. The protection scope of the present invention shall be subject to those defined by the appended claims.

In order to facilitate the understanding of the examples of the present invention, several specific examples in conjunction with the accompanying drawings will be taken as examples for further explanation and description, and the respective drawings do not constitute a limitation to the examples of the present invention.

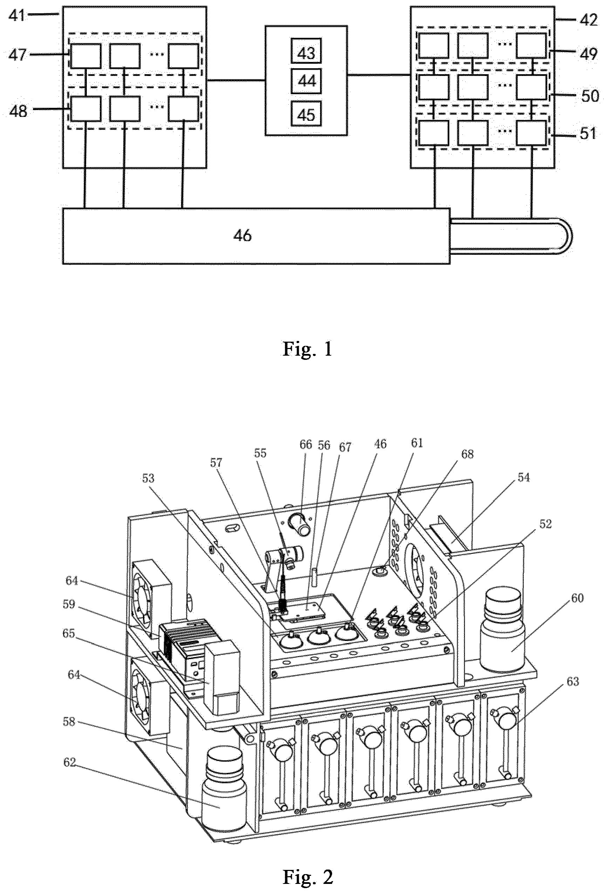

As shown in Figs. 1 and 2, the present invention relates to a microdroplet treatment device, which comprises: a sample introduction system, a microfluidic chip system, a temperature control system, a droplet recognition system, a droplet detection system, and a control system, wherein the sample introduction system is used for introducing a aqueous phase and an oil phase into the microdroplet treatment device, and the sample introduction system comprises at least: a sample introduction system I and a sample introduction system II, the sample introduction system I is used for introducing an oil phase into the microdroplet treatment device, and the sample introduction system II is used for introducing aqueous phase into the microdroplet treatment device; the microfluidic chip system comprises a substrate, pipes formed in the substrate, a first detection window and a second detection window; the temperature control system comprises a temperature sensor, and a temperature control component; the droplet recognition system comprises a laser light source and a photoelectric sensor; the droplet detection system comprises an optical fiber spectrometer and a halogen light source; and the control system is used for controlling each of the systems in the microdroplet treatment device.

Particularly, as shown in Fig. 1, the microbial droplet culture device according to the present invention comprises: a sample introduction system, a microfluidic chip system, a temperature control system, and a detection system (which may be a droplet recognition system and a droplet detection system), and a control system.

The sample introduction system consists of a sample introduction system I and a sample introduction system II, wherein the sample introduction system I is used for introducing an oil phase, and the sample introduction system II is used for introducing a aqueous phase; the sample introduction system I comprises a container bottle **a,** a sample introduction power source **a,** and conduits for interconnection; the sample introduction system II comprises a container bottle **b,** a sample introduction power source **b,** a container bottle **c,** and conduits for interconnection; wherein the container bottle **b** of the sample introduction system II contains a liquid which is incompatible and non-reactive with the container bottle **c** and also is incompressible, the introduction power source **a** drives the liquid in the container bottle **a** to enter the microfluidic chip system stably and controllably through the conduit; the bottom of the container bottle **c** contains the biological sample to be introduced, when the sample introduction power source **b** starts to drive, the liquid in the container bottle **b** enters the container bottle **c** through the conduit to increase the hydraulic pressure so that the biological sample at the bottom of the container bottle **c** enters the microfluidic chip stably and controllably; the power device is a sample introduction power source, preferably is a pressure pump or a injection pump.

In the device according to the present invention, the sample introduction system at least comprises two sample introduction systems I (sometimes referred to as oil phase sample introduction systems in the present invention) and one sample introduction system II (sometimes also referred to as aqueous phase sample introduction systems in the present invention), wherein the sample introduction system II comprises a liquid container, a power source, and a buffer bottle; the sample introduction system I comprises an oil phase container and a power source, wherein the power source is a injection pump, a peristaltic pump, a diaphragm pump and/or a plunger pump, preferably a injection pump; and wherein the liquid container contains a liquid which is immiscible with the sample solution to be introduced and is also incompressible, the power source drives the liquid into the buffer bottle via the input pipe, and the buffer bottle contains the sample solution; when the power source starts to drive, the liquid enters the buffer bottle via the input pipe to push the sample solution into the microfluidic chip system via the output pipe; wherein the liquid container, the power source and the buffer bottle are connected through the capillary pipe, and the oil phase container contains the medium oil.

In a specific embodiment, the sample introduction system in the device according to the present invention comprises three sample introduction systems I and three sample introduction systems II.

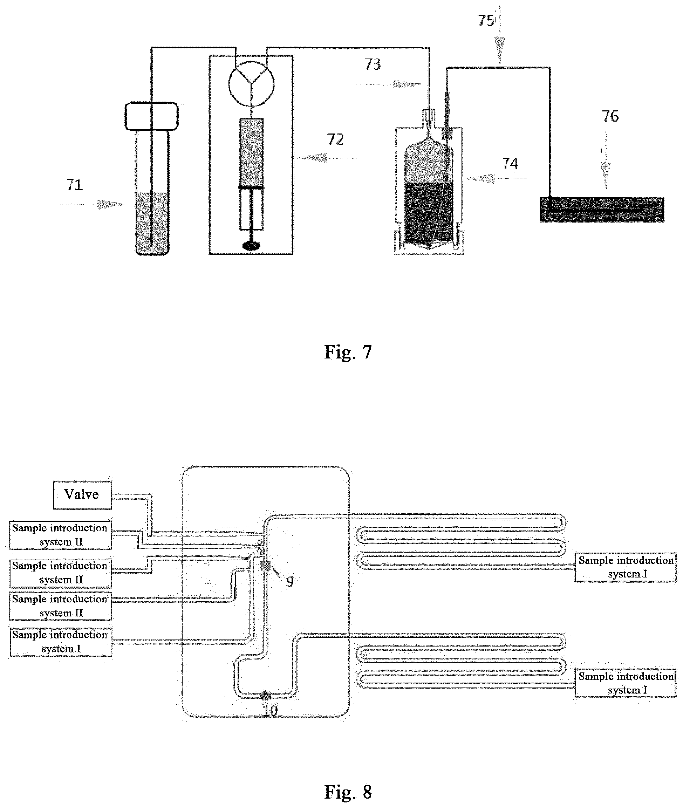

As an example of the sample introduction system II, it is shown in Fig. 7. It may be seen from Fig. 7 that the sample introduction system II comprises: a liquid container 71 (for example, the aforementioned container bottle **b**), which contains a hydraulic fluid that is incompatible with the sample solution and is incompressible, and the liquid container 71 is connected to the power source 72 via the hydraulic fluid output pipeline, the power source 72 drives the hydraulic fluid to enter the buffer bottle 74 via the input pipe 73. In the specific embodiment shown in Fig. 7, the density of the hydraulic fluid selected is lower than the density of the sample solution; the upper part of the buffer bottle 74 (for example, container bottle **c**) contains the hydraulic fluid, and the bottom part contains the sample solution, wherein the input pipe 73 is connected to the mouth of the buffer bottle 74, and the output pipe 75 is connected to the bottom of the buffer bottle 74. When the power source 72 drives, the liquid enters the buffer bottle 74 via the input pipe 73 to push the sample solution into the microfluidic chip system 76 via the output pipe 75. Here, the hydraulic fluid may be an oil phase. Among them, the output pipeline, the input pipe, etc. may all adopt the following capillary pipeline in the present invention.

In a specific embodiment, the above-mentioned liquid container is made of a rigid material, which may ensure that the liquid container has no flexibility change during the sample introduction process; wherein the rigid material is a plastic material or a metal material. The plastic material is any one or more selected from the group consisting of: PC (polycarbonate), ABS (acrylonitrile-butadiene-styrene copolymer), PMMA (polymethyl methacrylate), PS (polystyrene); the metal material may be a metal element, a metal oxide or a metal alloy, and the metal material is any one or more selected from the group consisting of iron, aluminum, copper, iron oxide, aluminum oxide, and copper oxide, and it also may be an alloy formed with any one or more of iron, aluminum, copper, iron oxide, aluminum oxide, and copper oxide, or an alloy formed with other materials.

The microfluidic chip system consists of a substrate, a microchannel formed on the substrate, a first detection window, and a second detection window, wherein the microfluidic chip system and the sample introduction system form a hermetic sterile structure via a pipeline.

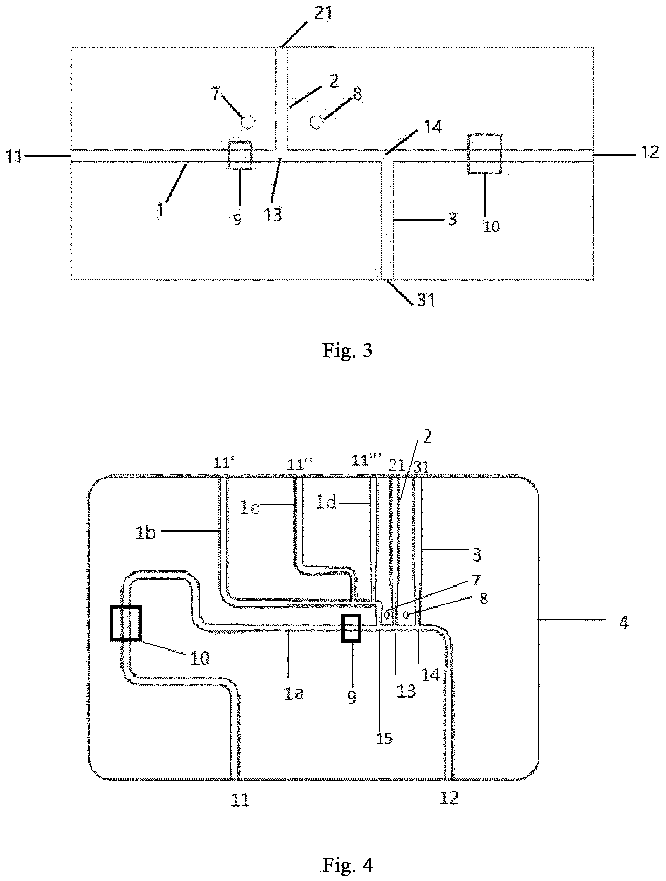

Fig. 3 and Fig. 4 respectively show an example of a functional schematic diagram and a specific structure diagram of the microfluidic chip used in the present invention. Fig. 3 shows a schematic structural diagram of a microfluidic chip according to the present invention, and the microfluidic chip at least comprises a substrate 4, and a first pipe 1, a second pipe 2 and a third pipe 3 formed on the substrate.

Particularly, in the microfluidic chip according to the present invention, the first pipe, the second pipe, and the third pipe formed in the substrate refer to the first pipe, the second pipe, and the third pipe formed inside the substrate.

The substrate 4 is a microfluidic chip substrate, which is made of material selected from the group consisting of: glass, polymethylmethacrylate (PMMA), polycarbonate (PC), polystyrene (PS), acrylonitrile-butadiene-styrene copolymer (ABS); wherein the first pipe 1, the second pipe 2, and the third pipe 3 are formed inside the substrate 4. In a specific embodiment of the present invention, the substrate and the pipe are formed together by carving.

In this embodiment, the pipe material is selected from the group consisting of: glass, polymethylmethacrylate (PMMA), polycarbonate (PC), polystyrene (PS), acrylonitrile-butadiene-styrene copolymer (ABS), preferably the constituent material is polymethyl methacrylate; the cross-sectional shape of the pipe is not limited, and it may be any shape convenient for forming and the flow of droplets, such as a circle, a rectangle, an oval, etc. The range of the cross-sectional area of the pipe is 2.5×10⁻³ mm²∼4 mm², preferably 0.01-3 mm², more preferably 0.1-2.5 mm², still more preferably 0.25-1 mm². It is further preferred that the cross-sectional area of the first pipe, the second pipe and the third pipe are the same. Those skilled in the art may reasonably set the thickness of the pipe according to the size of the chip substrate and the needs of the droplets to be cultured, detected and sorted.

In a specific embodiment, the cross-section of the pipeline according to the present invention is a square, and its side length has a range of 0.5-2 mm.

In a specific embodiment, the cross-sectional area of the first pipe, the second pipe, and the third pipe may be the same or different from each other. The first pipe, the second pipe, and the third pipe themselves may also be variable in diameter, i.e., the cross-sectional area of the first pipe, the second pipe, and the third pipe are not constant. It is applicable in the present invention, as long as the range of the cross-sectional area of the first pipe, the second pipe, and the third pipe satisfies the above-mentioned limitation.

As shown in Fig. 3, a first pipe 1, a second pipe 2, and a third pipe 3 are formed in the substrate 4; wherein the first pipe 1 comprises a first connection port 11 and a second connection port 12 at both ends, and the first pipe 1 may be used to accommodate the droplet **a** to be segmented and the droplet **b** to be fused before segmentation and fusion, and to accommodate the second portion droplet **a2** and new droplet **c** during segmentation and fusion. The second pipe 2 comprises a third connection port 21 at one end, and the other end communicates with the first pipe 1; wherein the second pipe 2 is used to accommodate the first portion droplet **al** after segmentation. The third pipe 3 comprises a fourth connection port 31 at one end, and the other end communicates with the first pipe 1, and the third pipe 3 is used to accommodate the second portion droplet **a2** after segmentation. The first communication part 13 of the first pipe 1 and the second pipe 2 is located upstream of the second communication part 14 of the third pipe 3 and the first pipe 1 in the moving direction of the droplet, and the distance between the first communication part 13 and the second communication part 14 is 500 µm∼2000 µm, preferably 750 µm∼1800 µm, more preferably 1000 µm∼1500 µm.

The distance between the first communication part 13 and the second communication part 14 is related to the size of the droplet and the cross-sectional area of the pipe. In the present invention, the distance is 500 µm∼2000 µm, preferably 750 µm∼1800 µm, preferably 1000 µm∼500 µm. Particularly, the distance may be 600 µm, 700 µm, 800 µm, 900 µm, 1100 µm, 1200 µm, 1300 µm, 1400 µm, 1600 µm, 1700 µm, or 1900 µm.

The chip according to the present invention also comprises a first detection window 9 and a second detection window 10 formed on the first pipe 1, as long as the first detection window 9 and the second detection window 10 may be used to realize the monitoring and detection of the microdroplets moving in the pipeline of the chip at this position. There is no restriction on the specific form of the detection window. If the chip and the pipe themselves are made from transparent material, the first detection window 9 and the second detection window 10 are two detection sites on the first pipe 1. If the material of the chip and the pipe itself is not transparent, two transparent parts need to be formed on the pipe itself to serve as the first detection window 9 and the second detection window 10.

In a specific embodiment, there is no specific limitation on the size of the first detection window 9 and the second detection window 10. Since the two detection windows are a section of the first pipe, the length of the section on the pipe may be, for example, 200 µm∼1 mm, preferably 500 µm∼1 mm. The detection window of such a length may monitor and detect the microdroplets moving in the pipeline more effectively and accurately.

When the chip is in use, and when microdroplets are transmitted inside the chip, the first detection window 9 and the second detection window 10 may be used as a droplet recognition point and a droplet detection point, for example, the droplet recognition point may detect whether the droplet passes the detection window based on a laser system, and the droplet detection point may be used, for example, to detect the spectral information of the droplet passing the detection window by a spectroscopy system.

The first pipe 1, the second pipe 2, and the third pipe 3 are formed in the chip substrate 4. The first connection port 11 and the second connection port 12 of the first pipe, and the third connection port 21 of the second pipe, and the fourth connection port 31 of the third pipe are all located at the edge of the substrate 4.

Of course, those skilled in the art may fully understand that Fig. 3 is only illustratively showing an example of the chip involved in the present invention, and any method known to those skilled in the art may be used to construct the components in the chip.

Particularly, the chip according to the present invention may be used to realize the segmentation and fusion operations of microdroplets. Fig. 3 also shows a connection structure for segmentation and fusion when a droplet **a** to be segmented and a droplet **b** to be fused enter the first pipe 1 from the first connection port 11.

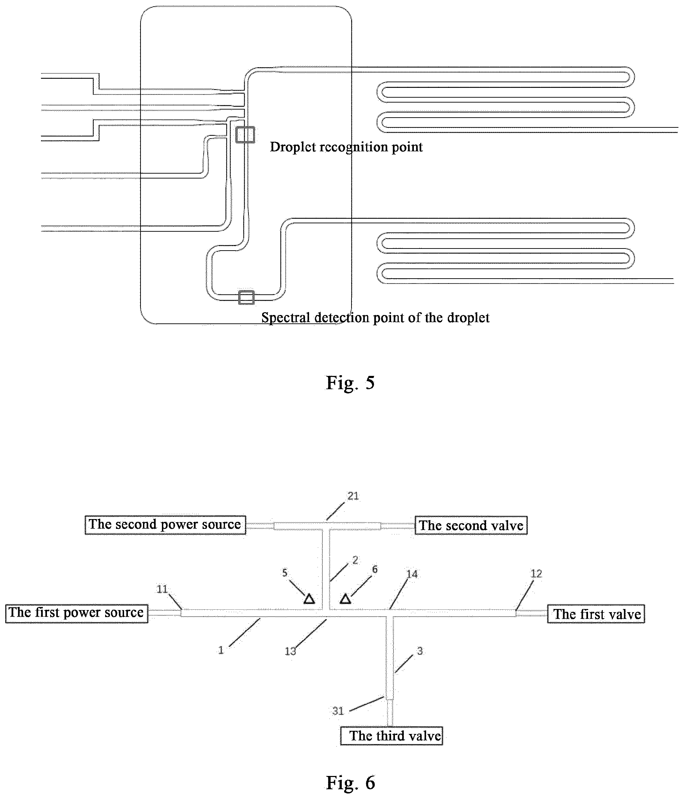

In a specific embodiment, for example, the first pipe 1 communicates with a first power source located outside the chip through the first connection port 11 and the capillary pipe, and the first pipe 1 communicates with the first valve located outside the chip through the second connection port 12 and the capillary pipe. The second pipe 2 communicates with the second power source located outside the chip and the second valve located outside the chip through the third connection port 21 and the capillary pipe respectively, and the third pipe 3 communicates with a third valve located outside the chip through the fourth connection port 31 and the capillary pipe. In this embodiment, one end of the capillary pipe is inserted into the connection ports of the first pipe, the second pipe, and the third pipe located at the edge of the substrate, and the other end is connected to the power source and the valve. The specific communication mode is shown in Fig. 6.

In the present invention, high-precision, smooth and pulsation-free liquid transmission may be realized by using a power source. In the specific embodiment of the present invention, the first power source or the second power source is any one independently selected from the group consisting of: a injection pump, a pressure pump, a peristaltic pump, a diaphragm pump, and/or a plunger pump, preferably the first power source and the second power source is a injection pump. In the present invention, the size of the range of the power source is not limited. Those skilled in the art may appropriately select a injection pump, pressure pump, peristaltic pump, diaphragm pump and/or a plunger pump with suitable range according to the amount of the sample to be introduced.

In the present invention, the pressure in each hermetic pipeline is changed by using a valve and by opening and closing the valve; and the flow direction of the droplets in each pipeline is controlled by the pressure change. In the present invention, the first valve, the second valve, or the third valve is any one independently selected from the group consisting of: a solenoid valve, a rotary valve, a rocker valve, and a pinch-off valve. Preferably, the first valve, the second valve, and the third valve are rotary valves.

In some specific embodiments, those skilled in the art may also understand that the valve may also be replaced by other forms of mechanisms or components. For example, a injection pump used as a power source may be used as a valve, as long as it may change the pressure in the hermetic pipeline by opening and closing.

In the present invention, the first pipe, the second pipe, and the third pipe are only used to indicate different types of pipes, and are not intended to limit the number of pipes. The first pipe may be several first pipes, the second pipe may be several second pipes, and the third pipe may be several third pipes.

In the present invention, the first valve, the second valve, and the third valve are only used to indicate valves with different functions, and are not intended to limit the number of valves. The first valve may be several first valves, the second valve may be several second valves, and the third valve may be several third valves.

In the present invention, the first power source and the second power source are only used to indicate power sources with different functions, and are not intended to limit the number of power sources. The first power source may be several first power sources, and the second power source may be several second power sources.

When the chip according to the present invention is in use, the chip may be connected to the power source and valve through the following capillary pipe. In a specific embodiment of the present invention, as described below, the cross-sectional area of the capillary pipe is in the range of 2.5×10⁻³ mm²∼4 mm², preferably 0.01-3 mm², more preferably 0.1-2.5 mm², still more preferably 0.25-1 mm².

The pipe connection port is connected with the capillary pipe, wherein the connection is hermetic, and then communicated with the power source and/or the valve through the capillary pipe. In order to ensure the stable conduction of the pressure of the power source, the capillary pipe is a hard tube, and the pipeline has no flexible changes. It is further preferred that the capillary pipe is any one selected from the group consisting of: a polytetrafluoroethylene tube (PTFE tube), a copolymer tube of perfluoropropyl perfluorovinyl ether and polytetrafluoroethylene (PFA tube), a polyether ether ketone tube (PEEK tube), a polycarbonate tube (PC tube), and polystyrene tube (PS tube).

In a specific embodiment of the present invention, the first pipe communicates with the first power source through the first connection port and the capillary pipe, and the first power source drives to generate pressure into the first pipe through the first connection port of the first pipe, thereby pushing the liquid **a** or the liquid droplet **b** into the first pipe from the first connection port, and controlling the movement of the liquid droplet in the pipe to perform droplet culture.

The second pipe communicates with the second valve through the third connection port and the capillary pipe, when the first power source drives and only the second valve is opened, the liquid droplet **a** in the first pipe may be controlled to flow into the second pipe; the third pipe communicates with the third valve through the fourth connection port and the capillary pipe, when the first power source drives and only the third valve is opened, the liquid droplet **a** in the first pipe may be controlled to flow into the third pipe. By alternately opening the second valve and the third valve, the segmentation of the droplet a may be completed.

The second pipe communicates with the second power source through the third connection port and the capillary pipe, when the second power source drives, pressure is generated into the pipe through the third connection port of the second pipe, thereby pushing the droplet **al** to be fused into the droplet **b** staying at the first communication part of the first pipe and the second pipe, and the fusion of the droplets is completed.

The first pipe communicates with the first valve through the second connection port and the capillary pipe, when the first power source drives and only the first valve is opened, the fused liquid **c** may be pushed to flow out of the second connection port.

The first pipe in the chip substrate is used to accommodate, for example, the droplet **a** to be segmented and the droplet **b** to be fused, and the droplet volume of the droplet **a** to be segmented and the droplet **b** to be fused is 0.5-10 µl, preferably 0.6-8 µl, more preferably 0.7-7 µl, still more preferably 0.8-6 µl, still more preferably 0.9-5 µl, still more preferably 1-3 µl.

In an embodiment of the present invention, as shown in Fig. 4, there are two holes (7, 8) on the chip substrate, and the holes (7, 8) are located adjacent to first communication part 13 of the first pipe 1 and the second pipe 2. The locations of the two holes are not fixed, and they may be located on both sides of the second pipe 2 or on both sides of the first pipe 1. The distance between the holes (7, 8) and the first communication part 13 of the first pipe 1 and the second pipe 2 is 0.1mm-1cm, preferably 0.3mm-5mm, more preferably 0.5 mm-2mm; and the holes (7, 8) are used to accommodate the additionally provided positive and negative poles of the fusion electrode, those skilled in the art may provid the positions of the holes (7, 8) within the above range according to the requirements of chip design. The frequency of the voltage loaded on the fusion electrode is 0-20000 Hz, preferably 1000-10000 Hz, and the electrode voltage is 1-5000V, preferably 500-1000V When the fusion electrode is connected to the power source, an electric field is generated to act on the droplet at the first communication part. The electric field may be either an alternating electric field or a constant electric field. The voltage applied by the electrode is 1-5000V, preferably 500-1000V. The application of such an electric field may further promote the fusion of the droplet **al** to be fused and the droplet **b** staying at the first communication portion 13 of the first pipe 1 and the second pipe 2.

In addition, in the present invention, the specific shape of the holes (7, 8) and the size of the holes are not limited, as long as they may be used to place an additionally arranged fusion electrode. A fusion electrode usually comprises positive and negative poles.

In the implementation process of those skilled in the art, according to the principle of the present invention, several first pipes, second pipes, and third pipes may be provided respectively on the chip for segmentation and fusion of different liquids, or the first connection port of the first pipe and the third connection port of the second pipe are expanded into several connection ports, which are respectively connected to different power sources to push different types of droplets a to be segmented and droplets **b** to be fused to flow into the first pipe.

By using several first pipes 1 and several second pipes 2, different types of droplets **a** to be segmented and droplets **b** to be fused may be pushed into the first pipe 1 or the second pipe 2 respectively. Those skilled in the art may design corresponding droplet segmentation and fusion devices by using the connection principle of the segmentation and fusion device of the present invention according to the requirements of droplet segmentation and fusion. Similarly, the first pipe 1a, the first pipe 1b, and the first pipe 1c, the second pipe 2a, the second pipe 2b, and the second pipe ac mentioned above are only illustrative, and the first pipe 1 may consist of **n** different sub-pipes, the second pipe may consist of **m** different sub-pipes, wherein **n** and **m** may be the same or different, and **n** and **m** may be independently selected from integers in the range of 1-20.

Similarly, the droplet **aa** to be segmented, the droplet **ab** to be segmented, the droplet **ac** to be segmented, and the droplet **ba** to be fused, the droplet **bb** to be fused, and the droplet **bc** to be fused are also illustrative. **n** first pipes and **m** second pipes may be used for the sample introduction of **n** different droplets **a** to be segmented and **m** different droplets **b** to be fused.

Furthermore, for **m** first pipes 1 and **n** second pipes 2, it is also possible to use **m** first power sources and **n** second power sources to control and push different droplets respectively. Of course, if the design is reasonable, it is possible to consider combining some of the power sources to realize the driving of **n** different droplets to be segmented and **m** different droplets **b** to be fused respectively.

In a specific embodiment of the present invention, the present invention relates to a microfluidic chip. As shown in Fig. 4, a first pipe 1a, a first pipe 1b, a first pipe 1c, a first pipe 1d, a second pipe 2, and a third pipe 3 are formed in the substrate 4; wherein the first pipe 1a comprises a first connection port 11 and a second connection port 12 at both ends; the first pipe 1b comprises a first connection port 11' at one end, and the other end merges with the first pipe 1a at the branching part 15; the first pipe 1c comprises a first connection port 11" at one end, and the other end merges with the first pipe 1a at the branching part 15; the first pipe 1d comprises a first connection port 11'" at one end, and the other end merges with the first pipe 1a at the branching part 15. The second pipe 2 comprises a third connection port 21 at one end, and the other end communicates with the first pipe 1, and the second pipe 2 is used to accommodate the first portion droplet **al** after segmentation. The third pipe 3 comprises a fourth connection port 31 at one end, and the other end communicates with the first pipe 1, and the third pipe 3 is used to accommodate the second portion droplet **a2** after segmentation. The first communication part 13 of the first pipe 1 and the second pipe 2 is located upstream of the second communication part 14 of the third pipe 3 and the first pipe 1 in the moving direction of the droplet, and the distance between the first communication part 13 and the second communication part 14 is 500µm∼2000µm, preferably 750µm∼1800µm, more preferably 1000µm∼1500 µm.

Further, the branching part 15 is located upstream of the first communication part 13, and the distance between the branching part 15 and the first communication part 13 is 500µm∼5000 µm, preferably 1000µm∼3000 µm, preferably 2000µm∼3000 µm.

In the above specific embodiment, the first detection window 9 and the second detection window 10 are formed on the first pipe 1a, as shown in Fig. 4.

As shown in Fig. 4, the first connection ports 11', 11", 11"', and the third connection port 21 and the fourth connection port 31 may be respectively connected to different power sources and valves for the sample introduction of oil phase and aqueous phase, wherein the sample introduction systems of the aqueous phase and the oil phase may be switched. For example, when the chip is just started up, the oil phase introduction system is connected to the above-mentioned connection port, so that the inside of the chip is filled with medium oil. When starting to introduce microbial droplets, part of the sample introduction system may start to introduce the aqueous phase, for example, it may be used to introduce microbial droplets for culture, enzyme reaction system for reaction, and fresh medium, chemical factors, and matrix reaction solution for culture, etc.

In a specific embodiment of the present invention, for example, the oil phase sample introduction system is connected to the first connection port 11'; the aqueous phase sample introduction system is connected to the first connection port 11"; the aqueous phase introduction system is connected to the first connection port 11"'; the aqueous phase sample introduction system is connected to the third connection port 21; and the fourth connection port 31 is connected to the valve system.

During the use of the chip and/or chip system according to the present invention, for example, the oil phase sample introduction system connected to the first connection port 11' passes the oil phase into the chip, and at the same time, the aqueous phase introduction system connected to the first connection port 11" pulses the aqueous phase sample into the chip at a given introduction time as required, thereby forming water-in-oil microdroplets, for example, a fresh medium to be used for inoculation of the bacterial solution, or a substrate solution containing the enzyme for reaction, at the connection between the first pipe 1b and the first pipe 1c, see Fig. 4 and Fig. 8.

The power source continues to push the above formed microdroplets to move towards the right to the connection between the first pipe 1d and the first pipe 1b. Then stop pushing the movement of the microdroplets, and the power source connected to the first connection port 11"' pushes another aqueous phase solution (for example, it may be a certain chemical factor solution for addition, or a solution containing another matrix for reaction) and the microdroplets stopped at the first pipe 1d and the first pipe 1b to form the droplet **b** to be fused as described above. Particularly, the droplet **b** to be fused may be a fresh medium added with a certain chemical factor, or a reactant solution combining different reaction matrices (or substrates).

When the chip is in use, as shown in Fig. 4, the oil phase (e.g., mineral oil used as a medium) is accommodated in the first pipe 1b, and the aqueous phase is accommodated in the second pipe 2, e.g., the bacterial liquid to be inoculated, or an enzyme solution to be reacted. A water-in-oil droplet is formed at the first communication part 13 of the first pipe 1a and the second pipe 2, i.e., the droplet **a** to be segmented, for example, a bacterial solution to be inoculated, or a solution containing an enzyme for reacting with reactant.

In the process of the chip and/or chip system being used, firstly, the droplets **a** to be segmented are formed, and then they are cultured in the first pipe, and then the above-mentioned structure of the present invention that may realize segmentation and fusion is used to form the droplets **b** to be fused; then the segmentation and fusion are performed, and the droplet **a** to be segmented is cut and fused to form a new droplet c. In addition, the second pipe 2 starts to accommodate the aqueous phase, and after the bacterial liquid has all formed into the water-in-oil droplets, the oil phase is refilled, and the second pipe is used as the second pipe described above for segmentation and fusion during the operation of the chip.

Therefore, as described above, Figs. 3 and 6 of the present invention show the basic chip structure for segmentation and fusion, and Figs. 4 and 8 show a specific embodiment of the chip and/or chip system according to the present invention. Those skilled in the art may understand that in the chip shown in Fig. 4, when its partial structure is the same as that of Fig. 3, the structure of the power source and valve may realize the structure shown in Fig. 6, i.e. the fusion and segmentation of droplets. When the chip shown in Fig. 4 is used for operation, whether it is to firstly form a droplet **a** to be segmented surrounded by medium oil in the chip, or to firstly form a droplet **b** to be fused surrounded by medium oil in the chip, the basic structure shown in Figs. 3 and 6 may be used to realize it, after implementation the chip shown in Fig. 4 may also use the basic structure shown in Figs. 3 and 6 to form a new droplet **c** surrounded by medium oil. By repeating the above process, several to hundreds of new droplets **c** may be formed.

Of course, the sample introduction system and valve system are connected by capillary pipes that are hermetically connected with the aforementioned sample introduction ports. As described above, typically, the sample introduction system mainly comprises a container for accommodating the liquid to be introduced, a pipeline for sample introduction, and a power source. The power source is, as described above, any one selected from the group consisting of: a injection pump, a pressure pump, a peristaltic pump, a diaphragm pump and/or a plunger pump; and preferably a injection pump. Also as described above, the valve may be any one selected from the group consisting of: a solenoid valve, rotary valve, rocker valve, and pinch-off valve, and preferably it is a rotary valve.

Those skilled in the art may understand that the above-mentioned sample introduction systems and valves connected to the first connection ports 11', 11", 11"', and the third connection port 21, and the fourth connection port 31 are only an example. Depending on the state of the chip, the relationship between these introduction systems and valves is replaceable.

In the present invention, it may also be considered that there is a culture system in the device, and the culture system consists of a capillary pipe connected with a microfluidic chip. For example, in the chip according to the present invention as shown in Fig. 4, the first connection port 11 is connected to the first culture pipeline through the capillary pipe, and the second connection port 12 is connected to the second culture pipeline through the capillary pipe. The first culture pipeline and the second culture pipeline may also consist of capillary pipes, and the length of the first culture pipeline and the second culture pipeline may be designed according to the requirements of the culture and the reaction system for the culture time and reaction time.

At the same time, by controlling the power source connected to the chip according to the present invention, the flowing direction of the microdroplets in the chip may be reversed. As mentioned above, the formed several to hundreds of new droplets **c** may be cultured in the first culture tube, and as required, the movement direction of the microdroplets may be reversed through the control of the power source, so that the reciprocating movement of the microdroplets in the pipeline is realized.

As shown in Fig. 4, in a specific embodiment, a first detection window 9 and a second detection window 10 are provided on the first pipe 1a on the chip according to the present invention. The description of the first detection window 9 and the second detection window 10 is as described above.

In addition, on the first pipe 1a, the distance between the first detection window 9 and the second detection window 10 is not limited, as long as the recognition and detection of the microdroplets flowing in the pipe may be realized respectively, for example, the distance between the first detection window 9 and the second detection window 10 (taking the distance along the pipeline as an example) may be 1cm∼10cm, preferably 3cm∼5cm. The first detection window 9 and the second detection window 10 may be respectively used as a recognition window and a detection window for microdroplets.

In a specific embodiment, the first detection window 9 is used as a recognition window for microdroplets, and the second detection window 10 is used as an optical detection window for microdroplets.

As mentioned above, the recognition of microdroplets may be achieved by cooperating with a laser system (for example, provided on the microdroplet treatment device according to the present invention) provided outside the chip. When the laser system continuously emits a laser beam to the first detection window 9, if a microdroplet passes through the first detection window 9, the laser beam will be temporarily blocked, and the external recording system may record the change of the laser beam.

The detection of microdroplets may be realized by cooperating with a spectroscopy system (for example, provided on the microdroplet treatment device according to the present invention) provided outside the chip. For example, a spectroscopic detector may be provided to detect absorbance value of the microdroplets passing through the second detection window 10, and for example, the absorbance value may reflect the degree of growth of microorganisms in a microorganism culture system, or reflect the color change of a reaction system with a color change.

Fig. 5 shows a schematic diagram of a microfluidic chip system according to the present invention, wherein the pipes outside the chip substrate in Fig. 5 represent capillary pipes. Similar to the structure shown in Fig. 4, wherein the first pipe branches into a first pipe **a,** a first pipe **b,** a first pipe **c,** and a first pipe **d** upstream of the first communication part; wherein the first detection window and the second detection window are formed on the first pipe **a,** and they are hermetically connected to the capillary pipe through the first connection port and the second connection port on the first pipe **a,** the first pipe **b,** the first pipe **c,** and the first pipe **d,** and hermetically connected to the capillary pipe through the third connection port and the fourth connection port of the second pipe and the third pipe respectively.

Wherein the range of the cross-sectional area of the first pipe, the second pipe, the third pipe, and the capillary pipe is 2.5×10⁻³ mm²∼4 mm², preferably 0.01-3 mm², more preferably 0.1-2.5 mm², still more preferably 0.25-1 mm²; and further preferably the cross-sectional area of the first pipe, the second pipe and the third pipe are the same. The hermetic connection part of the capillary pipe hermetically connected with the first pipe, the second pipe, and the third pipe is located inside the substrate.

In the present invention, there is no specific limitation on the above-mentioned hermetic connection. For example, after the chip is designed for structure, it is processed on an engraving machine, and then the chip substrate is pressed by a hot press, the capillary is inserted into the deep hole on the side of the chip, and glue is introduced to perform a hermetic bond.

The capillary pipe in the chip system according to the present invention is a rigid tube, and more preferably the capillary pipe is any one selected from the group consisting of: a polytetrafluoroethylene tube (PTFE tube), a copolymer tube of perfluoropropyl perfluorovinyl ether and polytetrafluoroethylene (PFA tube), a polyether ether ketone tube (PEEK tube), a polycarbonate tube (PC tube), and a polystyrene tube (PS tube).

Further, the temperature control system comprises a temperature sensor and a temperature control component; further, the temperature control component consists of a temperature rising component and a temperature lowering component, wherein the temperature rising component comprises a temperature controller and a metal bath, a container bottle and/or a hydraulic buffer bottle are placed in the metal bath, and the temperature controller has a fan.

In a specific embodiment of the present invention, the temperature rising component comprises a temperature controller and a metal bath, a container bottle and/or a hydraulic buffer bottle are placed in the metal bath, and the temperature controller has a fan; the temperature control system is also equipped with a temperature lowering device i.e., a fan, which is used to cool the operating environment of the equipment, and further cool the microorganism culture of the equipment.

In a specific embodiment of the present invention, the temperature rising component and the temperature lowering component may further comprise a water discharge temperature control system, and a water discharge temperature control plate is located in the microdroplet treatment device; wherein the water inlet and outlet of the water discharge temperature control plate are connected with an external thermostatic water bath equipment, and the temperature of the water discharge temperature control plate is controlled through the thermostatic mechanism of the thermostatic water bath equipment, so that the system is controlled for temperature rising and the temperature lowering, thereby achieving the effect of adjusting the temperature in the device.

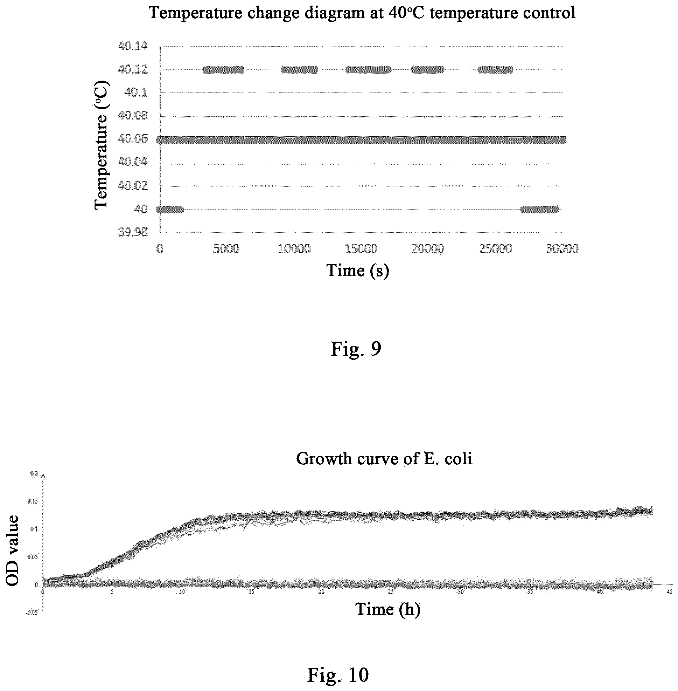

The temperature sensor may measure the temperature of the culture system according to the present invention online. The temperature control switch is turned on by the host computer software of the control system, the temperature control function and the fan are turned on by the circuit board program; after the temperature is detected, the stepwise heating is started; when the temperature is detected to reach the target value, some parameters are automatically adjusted, and the automatic control function for the fan is started with combination of the temperature sensor to keep the temperature between the target value (10-50)°C±0.2°C, wherein the temperature measured at 40°C are shown in Fig. 9.

In the present invention, it may be controlled to the generate 1-500 microdroplets, preferably 5-400 microdroplets, more preferably 10-300 microdroplets, still more preferably 20-200 microdroplets; wherein the volume of each microdroplet is 0.5-10 µL, and this has a far greater throughout than shaking bottles and deep-well plates and consumes less culture medium; it may realize regular and quantitative replacement of fresh culture medium and add chemical factors, and this is far more convenient than traditional seed solution subculture, thereby saving time and effort. Real-time online detection of the growth of microorganisms in each microdroplet is far more convenient than detection by traditional sampling, and the uninterrupted culture is truly achieved. Further, intelligently screened may also be performed by setting screening characteristics to automatically screen suitable strains, thereby improving efficiency.

Furthermore, the droplet recognition system comprises: a laser light source and a photoelectric sensor; and the droplet detection system comprises: an optical fiber spectrometer and a halogen light source.

Wherein the droplet recognition system recognizes the sample droplets of aqueous phase passing through the first detection window of the microfluidic chip system by using a photoelectric sensor and a laser light source; and the droplet detection system detects the spectral signal of the sample droplets of aqueous phase passing through the second detection window of the microfluidic chip system by using an optical fiber spectrometer and a halogen light source.

In an embodiment of the present invention, as shown in Fig. 2, the sample introduction system, the microfluidic chip system, the temperature control system, the droplet recognition system, and the droplet detection system are all arranged in a box, wherein the temperature rising component and the temperature lowering component of the temperature control system respectively adopt an air heater and a fan, and the air heater and fan are both provided inside the box; in another embodiment of the present invention (not shown in the Figure), the sample introduction system, the microfluidic chip system, the droplet recognition system, and the droplet detection system are provided in a box, wherein the temperature rising component and the temperature lowering component of the temperature control system respectively adopt water discharge temperature control system and a fan, and the water discharge temperature control plate is provided in the box, and the inlet and outlet of the water discharge temperature control plate are respectively connected with an external thermostatic water bath equipment, and the temperature of the water discharge temperature control plate is controlled through the external thermostatic water bath equipment, thereby adjusting the temperature in the box; and wherein the fan is provided in the box for lowering the temperature of the operating environment of the equipment.

The temperature inside the box is controlled by a temperature control system, and it is further preferred that the temperature inside the box is controlled within a range of 10°C∼50°C, and the range of temperature fluctuation is controlled within ±0.5°C. The box also comprises a sterilization device, and preferably the sterilization device is an ultraviolet lamp.

In the device according to the present invention, a control system is used to control each system in the microdroplet treatment device.

The control system comprises: a controller and a PC display control system; wherein the controller is respectively connected to the sample introduction system, the temperature control system, the droplet recognition system, and the droplet detection system, and is controlled by the digital circuit in the control system; and wherein the PC display control system displays, stores and analyzes the information of the sample introduction system, the microfluidic chip system, the temperature control system, the droplet recognition system, and the droplet detection system.

### EXAMPLE

### Example 1

The first pipe, the second pipe, and the third pipe are pipes formed in the chip substrate, wherein the size of the chip substrate is 3cm^{∗}5cm^{∗}4mm (length^{∗}width^{∗}thickness), and the material used for the chip substrate is PMMA. The first pipe, the second pipe, and the third pipe formed in the chip substrate are pipes with a square cross-section having a cross-sectional area of 1 mm² (with a side length of 1mm). The first pipe is connected with the second pipe and the third pipe respectively, wherein the first communication part of the first pipe and the second pipe is located upstream of the second communication part of the first pipe and the third pipe, and the distance between the first communication part and the second communication part is 1.5mm. The pipeline is full of oily medium.

The first connection port of the first pipe is connected to the injection pump A, the second connection port is connected to the first rotary valve, the third connection port of the second pipe is connected to the injection pump B and the second rotary valve, the fourth connection port of the third pipe is connected with the third rotary valve; wherein the pipes, injection pumps, and rotary valves are connected with each other by capillary pipe, and the inner diameter of the capillary pipe is 1.0mm and the material of the pipe is PTFE.

In this example, the injection pump A and the injection pump B in use are both industrial injection pumps, and the valve head of the injection pump is a three-way valve. The first valve, the second valve, and the third valve are all high-pressure two-way valves.

Starting the injection pump A, and opening the first rotary valve, then a 2µl volume of droplet **a** to be segmented and a 2µl volume of droplet **b** to be fused are pushed into the first pipe; the droplet **a** and droplet **b** are blocked by the oily medium as oil phase in the pipe. In this example, the droplet to be segmented is a microbial culture solution containing *E. coli* BL21, and the droplet b is a fresh LB medium. The medium as oil phase is mineral oil.

Turning off the first rotary valve, opening the second rotary valve, and continuing to drive the injection pump A, the droplet **a** is pushed to move to the first communication part of the first pipe and the second pipe, wherein 70% of **a** is droplet **al** (i.e., about 1.4µl) which enters the second pipe. At this time, turning off the second rotary valve, opening the third rotary valve, and continuing to drive the injection pump A, the remaining 30% of droplet **a** (about 0.6µl) as droplet **a2** all enter the third pipe, finally the droplet **a** is segmented into two partial droplets **al** and **a2;** the droplet **al** remains in the second pipe, and the other part (i.e., the droplet **a2**) enters the third pipe, then the third rotary valve is turned off and the segmentation of the droplet is completed. The segmentation of the droplet **a** is mainly to fit the droplet **a** so that the bottom surface of **a** is parallel to the pipe A, thereby reducing the amount of oil phase in the intermediate interval, and then quantitatively injecting part of the solution that fits the droplet A into the droplet **b** to be fused, so as to achieve high accuracy of the introduction volume. Since the droplet **a** is cut off by the oil phase in the pipeline, one end of the droplet **b** is flush with the first pipe channel, laying the foundation for subsequent accurate quantitative sample introduction.

Turning on the first rotary valve, continuing to drive injection pump A, droplet **b** is pushed to move to the junction of the second pipe and the first pipe, stopping the pushing, and then driving injection pump B to push part (**a1**) of droplet **a** quantitatively into droplet **b** to form a new droplet **c.**

Starting the injection pump A to push the new droplet c to move forward, the remaining droplet **al** partially resides in the channel B, thereby completing the quantitative exchange of the droplet **a** and the droplet **b.**

The above steps are repeated cyclically, so as to inoculate multiple culture solutions containing *E. coli* BL21 into fresh LB medium.

### Example 2

As shown in Fig. 4, the same material as in Example 1 is used to form a microfluidic chip. In Example 2, the length of the substrate is 7.5 cm, and the width is 5 cm.

In addition, in Example 2, the configuration of the sample introduction system is shown in Fig. 8. The microfluidic chip is connected with 3 sample introduction systems I and 3 sample introduction systems II, wherein the same methods as those in Example 1 are used for the power sources and valves.

In Example 2, before using the chip, it is filled with the oil phase by the sample introduction system I, and then the bacteria liquid for inoculation is introduced into the second pipeline 2 in a sterilized state by the sample introduction system II connected to the second pipeline 2 to form water-in-oil microdroplets at the first communication part 13. After completing the sample introduction, the second pipe 2 is again filled with oil phase. Next, being driven by the power source of the sample introduction system I, the water-in-oil microdroplets formed at the first communication part 13 reciprocate in the first pipe 1a to culture the bacteria in the droplets which becomes droplets **a** to be segmented after the culture; wherein the first detection window 9 is used as a droplet recognition point to identify the position of the droplet; and the second detection window 10 is used as a droplet detection point for detection of bacterial concentration (OD value).

Then, the chip is filled with the oil phase from the first connection port 11' by the sample introduction system I, and the culture medium of aqueous phase is intermittently filled into the chip from the first connection port 11' by the sample introduction system II through controlling time period of sample introduction as required, thereby forming a water-in-oil culture medium droplet at the connection between the first pipe 1b and the first pipe 1c. The following steps may be selected, when a microdroplet moves to the right to the connection between the first pipe 1d and the first pipe 1b, then stopping pushing the movement of the microdroplet; and the sample introduction system II connected to the first connection port 11"' pushes the sodium chloride solution into the medium droplet ceased and formed at the first pipe 1b and the first pipe 1d (change the concentration of the culture factor in the medium) to form the droplet **b** to be fused, which enters the first pipe and is located on the left side of the droplet **a** to be segmented, the liquid is pushed to move from the first connection port 11 to the second connection port 12 in the first pipe 1a.

The process of segmentation and fusion for the droplet **a** to be segmented and the droplet **b** to be fused is repeated the same as in the above Example 1. After completing the segmentation and fusion of all the droplets, a new droplet culture of microorganisms is performed again, and the OD value of the droplet may be detected again through the second detection window 10.

### Example 3

In Example 3, as shown in Fig. 2, the sample introduction system, the microfluidic chip system, the culture system, the temperature control system, the droplet recognition system, and the droplet detection system are all provided in a box. Through the setting mode shown in Fig. 2, the microfluidic chip system in Example 2 is adopted to realize the segmentation and fusion of droplet, and the cultivation of new microbial droplet.

As shown in Fig. 8, wherein the sample introduction system comprises three sample introduction systems I, three sample introduction systems II, valves and waste bottles. The sample introduction system I consists of a power pump and an oil phase bottle, and the sample introduction system II consists of a power pump, an oil phase bottle, and a sample buffer bottle, wherein the oil phase medium stored in the oil phase bottle used in the sample introduction system I is the same as the oil phase medium stored in the oil phase bottle in the sample introduction system II. In a specific embodiment, the oil phase bottle may be shared. In this example, as shown in Fig. 2, the three sample introduction systems I and the three sample introduction systems II share an oil bottle 60 to accommodate the oil phase medium, and the three sample introduction buffer bottles 61 accommodate the aqueous phase bacterial liquid, medium, and chemical factors for addition; six injection pumps 63 are the power source of the sample introduction system, and the six injection pumps 63 are all connected to the oil bottle 60, wherein three of them push the oil phase medium of the oil bottle 60 into the microfluidic chip 46, thereby forming three sample introduction systems I, as shown in Fig. 8; the other three injection pumps push the oil phase medium of the oil bottle 60 into the three sample buffer bottles 61 respectively, and the aqueous phase liquid in the sample buffer bottle 61 is pushed into the microfluidic chip 46 by the liquid pressure of the oil phase medium, thereby forming three sample introduction systems II, as shown in 8; the injection pump 63, the oil bottle 60, the sample buffer bottle 61, the rotary valve 65, the waste bottle 62, and the microfluidic chip 46 are connected by a capillary pipe (not shown in the figure) according to the connection mode of Example 2. Through the cooperation between the above-mentioned sample introduction systems and control of valves, the bacteria to be cultured are segmented and fused in the microfluidic chip system 46 to form water-in-oil microbial droplets which are driven to reciprocate and culture in the pipeline of the microfluidic chip system 46 through the function of the injection pump 63.

The temperature control system comprises a temperature rising component, a temperature lowering component, and a temperature control component. As shown in Fig. 2, the temperature rising component comprises an air heater 54 and a metal bath 53, the sample buffer bottle 61 is placed in the metal bath 53, and the air heater 54 heats up the culture environment for the microorganism in the box of the device by a heating fan; the temperature lowering component comprises two fans 64 which are used to cool the operation environment of the device and the culture environment of the microorganism respectively. The temperature of the culture environment of the microorganism in the box of the device is measured by the temperature probe 67, and fed back to the control system of the device so as to control the operation of the temperature rising component and the temperature lowering component, thereby heating the droplets in the microfluidic chip and controlling at a constant temperature. In this example, the temperature control curve is shown in Fig. 9.

The droplet recognition system comprises a laser light source 55 and a photoelectric sensor 56. As shown in Fig. 2, the laser light source 55 irradiates the first detection window of the microfluidic chip system 46, so as to recognize the aqueous phase sample droplet passing through the first detection window, and then the position information of the droplet is transmitted back to the control equipment of the device by the photoelectric sensor 56.

The droplet detection system comprises an optical fiber 57, a spectrometer 58, and a halogen light source 59. As shown in Fig. 2, the optical fiber 57 is aligned with the second detection window of the microfluidic chip system 46, and the spectral signal of the aqueous phase sample droplet passing through the second detection window of the microfluidic chip system is detected by the spectrometer 58.

In the detection system, the state of the microdroplet at the first detection window of the microfluidic chip system 46 is recognized by using the photoelectric sensor 56 in conjunction with the laser light source, and the microdroplet may be controlled to reciprocate circularly for culture. The optical fiber 57, the spectrometer 58, and the halogen light source 59 are used cooperatively to detect the sample in accommodated microdroplet at the second detection window of the microfluidic chip system 46. The EP tube 52 is used to accommodate the cultured microorganism droplets, and the ultraviolet lamp 66 may sterilize the microbial culture environment in the device.

The steps of microbial culture are as follows:
1) turning on the UV lamp and sterilizing the instrument for 30 minutes;
2) exhausting the pipeline of the device, turning on the temperature control system, and preheating for 30 minutes;
3) adding 1 mL of the inoculated bacterial solution (as described above, *E. coli* BL21) to an sample introduction buffer bottle, and putting it in a vacuum degassing box to perform degasification for 30 minutes; another sample introduction buffer bottle is filled with a fresh culture medium (LB medium) for cultivation; and still another sample introduction buffer bottle is filled with a solution of chemical factor for addition, i.e., ascorbic acid solution;
4) installing the chip on the bracket and connecting it with the inlets of the sample introduction buffer bottle and the oil bottle;
5) turning on the software of the instrument, selecting the "growth curve" measurement mode, and setting the basic parameters: the detection period is 0.5h, the number of droplets is 100; the detection wavelength is 620nm;
6) clicking to run;
7) the data is obtained after running for 46h, and the result is shown in Fig. 10.

The present invention may realize precise temperature control to make the results highly reliable, and the microorganisms may grow smoothly to realize continuous cultivation for up to 90 days.

### INDUSTRIAL APPLICABILITY

The fully automatic high-throughput microbial droplet culture device and use thereof according to the present invention may be manufactured and used in the field of high-throughput microbial culture.

Although the embodiments of the present invention are described above with reference to the accompanying drawings, the present invention is not limited to the above-mentioned specific embodiments and application fields. The above-mentioned specific embodiments are only illustrative, instructive, and not restrictive. Under the enlightenment of this specification and without departing from the protection scope of the appended claims of the present invention, those of ordinary skill in the art may also make many forms of modification which all belong to the protection scope of the present invention.

## Claims

1. A microdroplet treatment device, comprising: a sample introduction system, a microfluidic chip system, a temperature control system, a droplet recognition system, a droplet detection system and a control system; wherein
the sample introduction system is used for introducing samples of aqueous phase and oil phase into the microdroplet treatment device, and the sample introduction system comprises at least: a sample introduction system I and a sample introduction system II, the sample introduction system I is used for introducing samples of oil phase into the microdroplet treatment device, and the sample introduction system II is used for introducing samples of aqueous phase into the microdroplet treatment device;
the microfluidic chip system comprises a substrate, pipes formed in the substrate, a first detection window and a second detection window;
the temperature control system comprises a temperature sensor and a temperature control component;
the droplet recognition system comprises a laser light source and a photoelectric sensor;
the droplet detection system comprises an optical fiber spectrometer and a halogen light source; and
the control system is used for controlling each of the systems in the microdroplet treatment device.

2. The device according to claim 1, wherein
the droplet recognition system recognizes the sample droplets of aqueous phase passing through the first detection window of the microfluidic chip system by using the photoelectric sensor and the laser light source;
the droplet detection system detects the spectral signal of the sample droplets of aqueous phase passing through the second detection window of the microfluidic chip system by using the optical fiber spectrometer and the halogen light source.

3. The device according to claim 1 or 2, wherein
the sample introduction system at least comprises two sample introduction systems I and one sample introduction system II.

4. The device according to claim 3, wherein
the sample introduction system comprises three sample introduction systems I and three sample introduction systems II.

5. The device according to claim 3 or 4, wherein
the sample introduction system I comprises a liquid container and a power source;
the sample introduction system II comprises a liquid container, a power source, and a buffer bottle;
the power source is a injection pump, a peristaltic pump, a diaphragm pump, and/or a plunger pump, preferably the injection pump;
the liquid container contains a liquid immiscible and incompressible with the sample solution to be introduced;
the power source drives the liquid entering the buffer bottle via an input pipe;
the buffer bottle contains the sample solution, when the power source drives the liquid, it enters the buffer bottle via the input pipe to push the sample solution into the microfluidic chip system via an output pipe, and wherein the liquid container, the power source and the buffer bottle are connected via a capillary pipe.

6. The device according to any one of claims 1-5, wherein
the microfluidic chip system comprises:
the substrate; and
a first pipe, a second pipe, and a third pipe formed in the substrate, wherein
the first pipe comprises a first connection port and a second connection port at both ends,
the second pipe comprises a third connection port at one end, and communicates with the first pipe at the other end, and
the third pipe comprises a fourth connection port at one end, and communicates with the first pipe at the other end,
the first communication part of the first pipe and the second pipe is located upstream of the second communication part of the third pipe and the first pipe in the direction of droplet movement, and the distance between the first communication part and the second communication part is 500 µm -2000 µm, preferably 750 µm-1800 µm, more preferably 1000 µm-1500 µm,
the first detection window and the second detection window formed on the first pipe,
the first detection window and the second detection window are transparent areas formed on the first pipe, and
a capillary pipe hermetically connected with the first pipe, the second pipe, and the third pipe.

7. The device according to claim 6, wherein
the first pipe is branched into a first pipe **a,** a first pipe **b,** a first pipe **c,** and a first pipe **d** upstream of the first communication part,
wherein the first detection window and the second detection window are formed on the first pipe **a,**
the first connection port and the second connection port on the first pipe **a,** the first pipe **b,** the first pipe **c,** and the first pipe **d** are hermetically connected with the capillary pipe,
the second pipe and the third pipe are hermetically connected to the capillary pipe through the third connection port and the fourth connection port respectively.

8. The device according to claim 6 or 7, wherein
the substrate and the first pipe, the second pipe and the third pipe are made of glass, polymethylmethacrylate (PMMA), polycarbonate (PC), polystyrene (PS), or acrylonitrile-butadiene-styrene copolymer (ABS); preferably polymethylmethacrylate (PMMA);
the capillary pipe is a rigid tube, and more preferably the capillary pipe is any one selected from the group consisting of: a polytetrafluoroethylene tube (PTFE tube), a copolymer tube of perfluoropropyl perfluorovinyl ether and polytetrafluoroethylene (PFA tube), a polyether ether ketone tube (PEEK tube), a polycarbonate tube (PC tube), and a polystyrene tube (PS tube); and
more preferably the range of the cross-sectional area of the first pipe, the second pipe, the third pipe and the capillary pipe is 2.5×10⁻³ mm²-4 mm², preferably 0.01-3 mm², more preferably 0.1-2.5 mm², still more preferably 0.25-1 mm².

9. The device according to any one of claims 1-8, wherein the sample introduction system and the microfluidic chip system are connected by capillary pipes and connection ports to form a hermetic closed-loop control structure.

10. The device according to claim 1, wherein
the sample introduction system, the microfluidic chip system, the droplet recognition system, and the droplet detection system are all provided in a box, and
the temperature inside the box is controlled by the temperature control system,
preferably the temperature inside the box is controlled within the range of 10°C-50°C, and the range of temperature fluctuation is controlled within ±0.5°C.

11. The device according to claim 10, wherein the box further comprises a sterilization device, preferably the sterilization device is an ultraviolet lamp.

12. The device according to any one of claims 1-11, wherein
the control system comprises a controller and a PC display control system;
the controller is respectively connected to the sample introduction system, the temperature control system, the droplet recognition system, and the droplet detection system, and takes control via a digital circuit in the control system;
the PC display control system displays, stores and analyzes the information from the sample introduction system, the microfluidic chip system, the temperature control system, the droplet recognition system, and the droplet detection system.
